# EUROPEAN PATENT APPLICATION

(11) **EP 4 635 397 A1**
(43) Date of publication of application: **22.10.2025**
(21) Application number: 25169552.4
(22) Date of filing: 09.04.2025
(51) Int. Cl.: A61B 1/012

(54) **PROTECTIVE DEVICE FOR AN ENDOSCOPIC APPARATUS**

(30) Priority: 16.04.2024 IT 202400008575
(71) Applicant: Bidoia Medica Sas Di Bidoia Gianfranco, 35010 Vigonza (PD) (IT)
(72) Inventor: BIDOIA, Gianfranco, 35142 Padova (IT)
(74) Representative: Modiano, Micaela Nadia

(57) **Abstract**

A protective device (10) for an endoscopic apparatus comprising a handpiece (11) made of plastic material from which a self-supporting, rigid cannula (12) made of metallic material protrudes, the handpiece (11) comprising two tubular and flexible appendages (17a, 17b) which converge into a primary conduit (24) which passes through the handpiece (11) internally, and further comprising at least one three-way valve (25a, 25b) engaged with at least one of the flexible tubular appendages (17a, 17b).

## Description

The present invention relates to a protective device for an endoscopic apparatus.

The protective device according to the invention can be applied in the medical sector, especially in the field of diagnostics and of endoscopic surgery, in particular in gynecology and urology; however, it can also be applied in other branches of medicine, such as for example laparoscopy.

Nowadays diagnostic and surgical techniques are increasingly widespread which involve the use of endoscopic apparatuses provided with optical monitoring systems and instruments of extremely reduced dimensions, even in the order of millimeters.

In the specific case of endoscopic operations, it is necessary to use instruments that are minimally invasive for the patient but which, at the same time, enable the medical surgeon to operate and to monitor the operation, for example by way of optical systems that make it possible to display the affected region.

Normally, these apparatuses comprise a probe comprising a shaft containing a bundle of optic fibers and an end optical element, generally a lens, which is adapted to be inserted into the body of the patient.

Generally this probe is connected, at an opposite end from the end with the optical element, to an image display and acquisition system, such as, for example, a video camera.

In some applications, the endoscopic apparatus involves the use of a cannula, for containing the probe, which serves to direct the probe toward the target organ.

In this type of apparatus, the connecting element between the cannula and the image acquisition system is tubular in extension and comprises a plurality of openings that are interfaced: to a lighting system; to one or more systems for administering fluids, such as physiological saline or sterilized water, which are controlled by valves; to one or more entry routes for the insertion of surgical or diagnostic instruments, such as for example a biopsy probe.

Such systems and instruments use the interspace generated between the internal wall of the cannula and the optical endoscopic probe in order to reach the target organ.

In order to ensure the hygiene and health security of the patient subjected to the examination and/or surgery, it is necessary for all instruments and/or apparatuses that are used and which come into contact with the human body to be sterile.

Such instruments can be of two types, based on the materials of which they are made:
- completely made of metal, the components of which are reusable, washable and repeatedly sterilizable using known and standardized methods,
- completely made of plastic material or with some components of metal, in any case single-use and already rendered sterile, using known and standardized methods.

For endoscopy instruments made entirely of metal, the end of the cannula is open, in order to allow the insertion into the body of other instruments, called surgical instruments, or the inside of the body to be viewed, up to the target organ, using optical devices like the probe.

For the instruments to be minimally invasive, the diameters of the tubes carrying the optical devices, which must ensure the illumination of the area to be operated on or examined and at the same time provide the image of that area to the monitor, are inherently very small and extend for lengths that can reach 310 mm. These parts are therefore thin and long, and for this reason they are delicate and sensitive to repeated cleansing, washing and sterilization cycles, which is why they are inserted in parts that are more resistant to sterilization, like the cannula. The cannula, of metal or of plastic material, which comes into contact with the patient and with his or her bodily fluids, must be sterilized after every single use.

The sterilization of surgical and/or monitoring instruments requires a considerable expenditure of time, which creates a block of time during which these instruments cannot be used, resulting in long waits and, as a consequence, the impossibility of performing examinations and/or operations in an efficient manner.

Furthermore, the sterilization of individual instruments brings high costs, not only for labor but also for purchasing the substances necessary to ensure adequate cleaning.

Instruments made at least partially of plastic material are typically single-use, sometimes even single-patient, and these must also be sterile in order to come into contact with parts subjected to surgery or in order to enter existing cavities of the body or parts that are to be subjected to operations, such as biopsies, diathermocoagulations or other operations that can cause bleeding, including when the target organs are hard to reach owing to the difficulty of introducing the instruments into cavities that are problematic to penetrate.

To this end, apparatuses are nowadays often used with a non-single-use part, for example the optical instrumentation, and a single-use part. In apparatuses for endoscopy, these are typically single-use kits which have an external cannula with a sheath inside it, both of which are made of plastic material. The sheath has a free end which is adapted to be inserted into the patient, where the optical element of the probe reaches. The closing section of the sheath, at the free end, normally has a beaker-shaped optical element, inserted inside or outside the free end of the sheath and heat-sealed or glued to it. The optical probe is inserted into the sheath.

This sheath ensures that the probe never comes into contact with the patient and with his or her bodily fluids. It is possible to simply replace the kit after each use of the apparatus without needing to sterilize the optical probe, thus reducing the running costs of the apparatus, the time not in use, and the wear of the optical probe.

This conventional technique shows room for improvement, however.

Normally the optical probe comprises an end element which is provided with a lens inclined at a 30° angle, and the sheath is made of nonrigid plastic material with a certain capacity for stretching, such as for example polyurethane, which can easily break during the introduction and/or rotation of the probe.

Furthermore this sheath is shorter than the probe, so that it stretches in length upon the passage of the probe, in so doing thinning the wall and so determining a better flow of light and of images, but the inclined lens often ends up perforating the sheath.

Furthermore, similar probes can have different lengths, both because each maker can vary the lengths from a few tenths of a millimeter to more than a millimeter, and also because any repairs can entail a reduction in length of as much as 4 mm.

These drawbacks have been overcome in the manner taught in Italian patent document no. 102017000106689 filed on 25 September 2017 by the same applicant, wherein the sheath is made of self-supporting plastic material, thus preventing breakages deriving from the use of a sheath of nonrigid material, but it is still adaptable, allowing a partial elastic lengthening.

Such known art also has a number of drawbacks, however.

Probes are, in fact, fragile and delicate and repeated cycles of cleaning and sterilization, as well as being handled by operators, can compromise their robustness.

Probes are in fact introduced into difficult cavities, such as bladder, cardiac, uterine, vaginal and/or cranial cavities, and they can be used to dilate parts inside the body by conveniently directing the cannula and displacing internal tissues and/or organs, sometimes stenotic, causing significant bending which can result in the breakage of the shaft of the probe.

A particular type of device overcomes the abovementioned drawbacks by introducing, within a handpiece, an elastically extensible element, partially made of plastic material and coupled to a self-supporting rigid cannula which protrudes from such handpiece.

The presence of the elastically extensible element makes it possible to easily compensate the different lengths of the various optics that can be used, without interfering in the transmission of light and images.

However, this prior art has a number of aspects that show room for improvement.

The conventional device has, in fact, two so-called "two-way" valves, which are associated with interference with two flexible appendages which protrude from its handpiece; these valves each have a first portion, adapted to be engaged with the flexible appendages, and a second portion, opposite from the first portion, specifically an inlet/outlet, adapted for the insertion of surgical instruments or for connection with one or more irrigation systems and/or systems for extracting fluid in the cavity targeted by the examination and/or by the surgery, such as physiological saline solutions or sterilized water.

The use of two-way valves places limitations on using the surgical instruments necessary for the operation.

In particular, the health operator can work with one surgical instrument at a time, and therefore must extract the instrument in use before he or she can insert and then use a different instrument for a subsequent step of the operation.

For example, in the case of removal of a polyp in the uterine cavity, the surgeon needs to introduce the scissors, cut the polyp, and then extract the scissors before he or she can introduce a surgical clamp to extract the polyp, or, for example, a coagulant electrode to coagulate the blood and prevent it from contaminating the washing liquid.

The need to introduce and subsequently extract multiple surgical instruments without the possibility of using them simultaneously, in addition to rendering the surgery quite difficult, inevitably requires more time than would be sufficient if it were possible to operate with multiple surgical instruments simultaneously.

Furthermore, the shape structure of the conventional devices means it is not possible to simultaneously perform the full extraction and irrigation of the washing fluid.

In fact, with these instruments it is possible to irrigate the washing liquid through a two-way valve associated with one of the two flexible appendages, but only to partially extract it using the other appendage and using a second two-way valve, making use of the remaining space between the surgical instrument and the conduit into which it is inserted.

The aim of the present invention is to provide a protective device for an endoscopic apparatus which is capable of improving the known art in one or more of the aspects complained of above.

Within this aim, an object of the invention is to provide a protective device that allows the simultaneous insertion of multiple surgical instruments.

Another object of the invention is to provide a protective device that enables the simultaneous extraction and irrigation of a washing fluid in the cavity affected by the medical examination and/or by the surgical operation, even simultaneously with the insertion of multiple surgical instruments.

A further object of the invention is to provide a protective device that is capable of protecting the shaft of the optical probe from breakage owing to excessive bending and at the same time is capable of compensating for different probe lengths.

Another object of the invention is to provide a protective device that is single-use, by virtue of which a bending of the optical probe that could lead to the loss of the image is prevented.

Another object of the invention is to provide a protective device by virtue of which lengthy and expensive procedures to clean and sterilize the probe, which moreover could lead to damage to the apparatus, are not necessary.

Another object of the invention is to provide a protective device that does not impede and does not distort the transmission of light and of images.

Furthermore, the present invention sets out to overcome the drawbacks of the background art in a manner that is alternative to any existing solutions.

Another object of the invention is to provide a protective device that is highly reliable, easy to implement and low-cost.

This aim and these and other objects which will become more apparent hereinafter are achieved by a protective device of an endoscopic apparatus comprising a handpiece made of plastic material from which a self-supporting, rigid cannula made of metallic material protrudes, said handpiece comprising two tubular and flexible appendages which converge into a primary conduit which passes through said handpiece internally,
characterized in that it comprises at least one three-way valve engaged with at least one of said flexible tubular appendages.

Further characteristics and advantages of the invention will become more apparent from the description of a preferred, but not exclusive, embodiment of the protective device according to the invention, which is illustrated, by way of non-limiting example, in the accompanying drawings wherein:

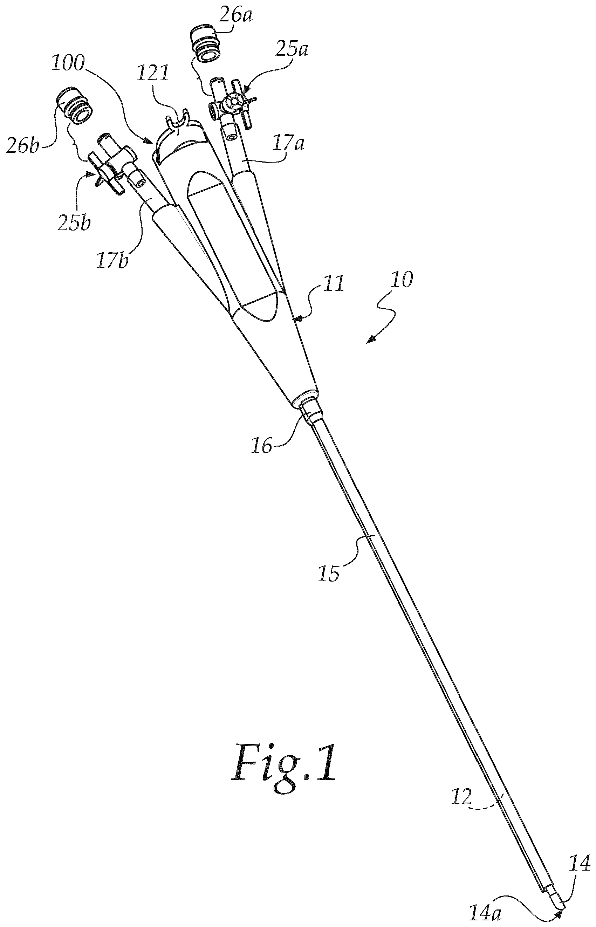

Figure 1 is an overall perspective view of the protective device for an endoscopic apparatus according to the invention;
Figure 2 is a partial cross-sectional view of the device shown in Figure 1;
Figure 3 is an overall perspective view from the front of a three-way valve of the device shown in Figure 1;
Figure 4 is an overall perspective view from below of a three-way valve of the device shown in Figure 1;
Figure 5 is a partial cross-sectional view of the three-way valve shown in Figures 3 and 4;

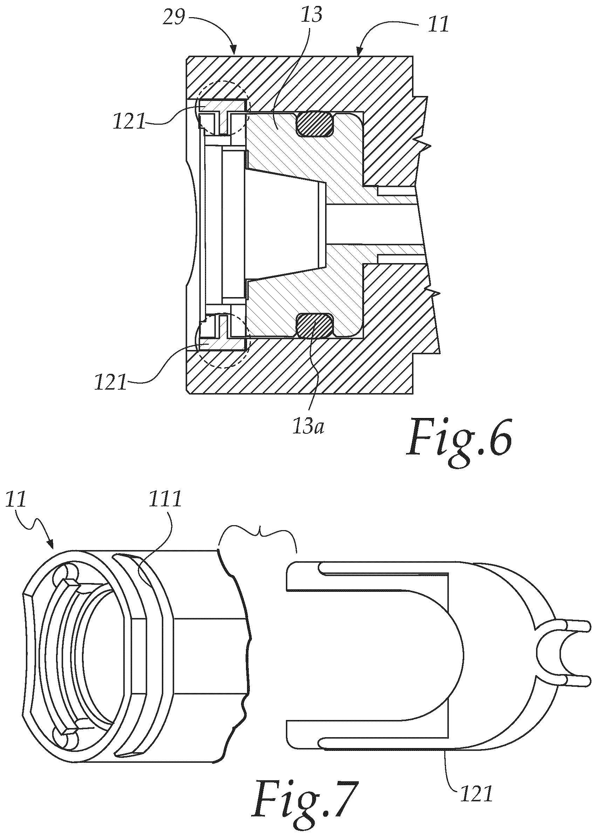

Figure 6 is a cross-sectional view of a first detail of the device shown in Figure 1;
Figure 7 is an exploded view of a second detail of the device shown in Figure 1;
Figure 8 is a partial perspective view of the device shown in Figure 1 in a configuration of use thereof.

With reference to the figures, the protective device for an endoscopic apparatus is generally designated by the reference numeral 10 and comprises a handpiece 11 made of plastic material from which a self-supporting, rigid cannula 12 made of metallic material protrudes.

The handpiece 11 comprises two tubular and flexible appendages 17a and 17b which converge into a primary conduit 24 which passes through the handpiece 11 internally.

According to the invention, the protective device 10 is characterized in that it comprises at least one three-way valve 25a, 25b engaged with at least one of the appendages 17a, 17b.

The handpiece 11 enables the healthcare worker to maneuver the endoscopic apparatus.

This handpiece 11 has an elongated box-like shape and from it, at one end, protrudes a self-supporting and rigid cannula 12, made of metallic material which is preferably stainless steel.

Inside the handpiece 11, the cannula 12 is joined to a part 29 with a through hole which is at least partially made of elastically extensible plastic material.

In particular, such part 29 comprises an end element 13 with a through hole and a sleeve 18 for connection between the cannula 12 and the end element 13.

The sleeve 18 is made of elastically extensible plastic material, preferably silicone rubber, and the end element 13 is inserted into an end section of the handpiece 11 which is conveniently contoured and protrudes partially therefrom.

In an alternative version, not shown, the part 29 can comprise an end portion with a through hole and a tubular portion that extends from the end portion and at which the cannula 12 is coupled; the end portion and the tubular portion are made in a single piece from elastically extensible plastic material.

Substantially, the end portion and the tubular portion can be identified with the previously-mentioned "end element 13" and "sleeve 18", but made in a single piece and of a single material, preferably silicone rubber.

By "self-supporting and rigid" what is meant is that the cannula 12 is capable of supporting itself and is not flexible, but tends instead to maintain its straight shape if subjected to bending forces.

In the handpiece 11, through the end element 13, the probe can be inserted of a system for acquiring and viewing images, not shown in the figures, and also a lighting device, also not shown in the figures.

The handpiece 11 advantageously has an upper opening 111, at an end portion 100 of the handpiece 11 that is opposite the one from which the cannula 12 protrudes.

The opening 111 is defined correspondingly to the end element 13, and has an elongated shape extending in a direction substantially perpendicular to the longitudinal direction of extension of the handpiece 11.

The end portion 13 is geometrically contoured for its form-fit coupling with a U-shaped element 121 adapted to be fitted externally onto the end portion 13 through the opening 111.

More specifically, the form-fit coupling between the end portion 13 and the element 121 is useful for fixing the optical probe, once the latter is inserted inside the device 10: the element 121 is in fact used to block the relative movement between the optical probe and the device 10 proper, so as to easily position the optical probe in the correct position.

The system for acquiring and viewing images is connected at the end of the handpiece 11, where inside it the end element 13 which acts as a connector is arranged, conveniently with a gasket 13a, preferably an O-ring.

At the free end of the cannula 12 there is a beaker-shaped optical end element 14 which closes the cannula 12 at the distal end of the device.

By the term "free end" what is meant is the end arranged opposite from the one inside the handpiece 11.

By the term "distal end" what is meant is the end of the device that is farthest from the operator, therefore opposite from the handpiece 11 and proximate to the optical end element of the probe.

The optical end element 14, which is fitted over and glued to the cannula 12, is made of transparent plastic material and has a base 14a with an inclination comprised between 0° and 30° with respect to the plane perpendicular to the plane of the walls and, preferably, 30°.

The device 10 also comprises a tubular covering 15 outside the cannula 12, which extends starting from the handpiece 11, where it is fitted with interference over a perforated shank 16 of that handpiece 11, toward the free end of the cannula 12, therefore toward the optical end element 14.

The cannula 12, after insertion of the optical probe, is adapted to protrude with respect to the covering 15.

The outer covering 15 is flexible and is made of plastic material, for example crystalline vinyl material.

In the example of the protective device 10 shown, the handpiece 11 comprises two tubular and flexible connecting appendages 17a and 17b, for the insertion of a system for administering fluids and/or for the insertion of surgical instruments.

The sleeve 18 for connection between the cannula 12 and the end element 13 is fitted over a perforated shank 19 of the latter.

Substantially the end element 13 presents a perforated flanged portion, on one side of which the vision system and the lighting device can be associated, and the perforated shank 19 extends on the opposite side from this portion. A single hole passes through them both in order to permit the passage of the probe.

At least one portion of the shank 19 is inserted in the sleeve 18 with interference at one end, and an end portion of the cannula 12 is inserted in the sleeve, again with interference, at the opposite end.

The device 10 can also comprise a band around the superimposed interference-coupled parts of the sleeve 18 and of the cannula 12; this band is made of heat-shrink material, to better ensure the adherence of the two underlying elements after its application.

The shank 19 of the end element 13 and the sleeve 18 are coupled by interference and with the interposition of adhesive, which is constituted preferably by a cyanoacrylic glue.

At the other end, the sleeve 18 and the cannula 12 are coupled by interference and with the interposition of another adhesive, which is constituted preferably by epoxy glue.

The two glues, cyanoacrylic and epoxy, can be mixed together for one or for both of the adhesive bonding seats.

The cannula 12, which, as has been mentioned above, is made of metallic material, in order to improve the grip of the glue thereupon, has a rough outer surface in the coupling region, preferably knurled, as well as a camber angle at its end, in order to facilitate the introduction of the optical probe.

In place of adhesives, the couplings between the same parts can be obtained via the use of ultrasound systems.

The sleeve 18 can in fact be coupled to the end element 13 and to the cannula 12 with joining techniques without glues, such as ultrasound, heat-sealing and the like.

The two appendages 17a and 17b merge in a conventional manner into a primary conduit 24 that passes through the handpiece 11 and the covering 15.

In particular, such appendages 17a and 17b are identical and mirror-symmetrical and they extend along convergent coplanar directions, defining two secondary conduits 27a, 27b which converge in a common zone with the primary conduit 24.

The cannula 12 acts as a guide both for the probe and for at least one surgical instrument, inserted from one of the appendages, toward the target organ.

The secondary conduits 27a and 27b lead inside the handpiece 11, in the primary conduit 24, so as to dispense inside the covering 15 the fluids used to actuate the apparatus and, at the same time, so as to allow the insertion of one or more surgical instruments.

The play deriving from the difference between the outside diameter of the cannula 12 and the inner diameters first of the handpiece 11 and then of the outer covering 15 determines the formation of an interspace that guides the fluids and/or the surgical instruments to the target organ, proximate to the optical element 14.

Each one of the appendages 17a and 17b is fitted with a "three-way" valve 25a or 25b, respectively a first valve 25a and a second valve 25b, inserted with interference into an end portion 40a or 40b of such appendages 17a and 17b and opposite from the end inserted in the handpiece 11.

The material of the appendages 17a and 17b and the material of the valves 25a and 25b are such as to provide a hermetic coupling, capable of preventing the accidental escape of the valves 25a and 25b from the appendages 17a and 17b and capable of preventing leaks of fluid.

In particular, the valve 25a or 25b is conventional.

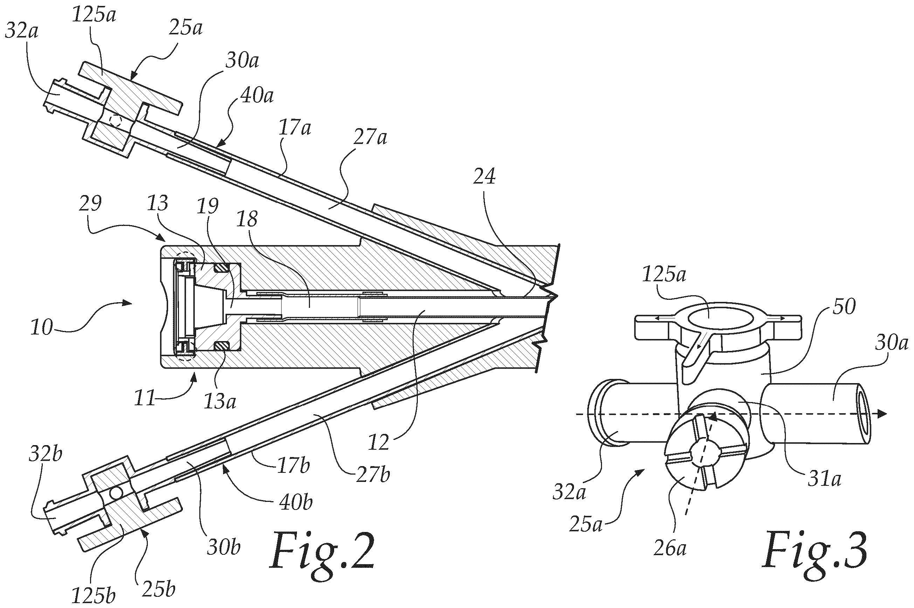

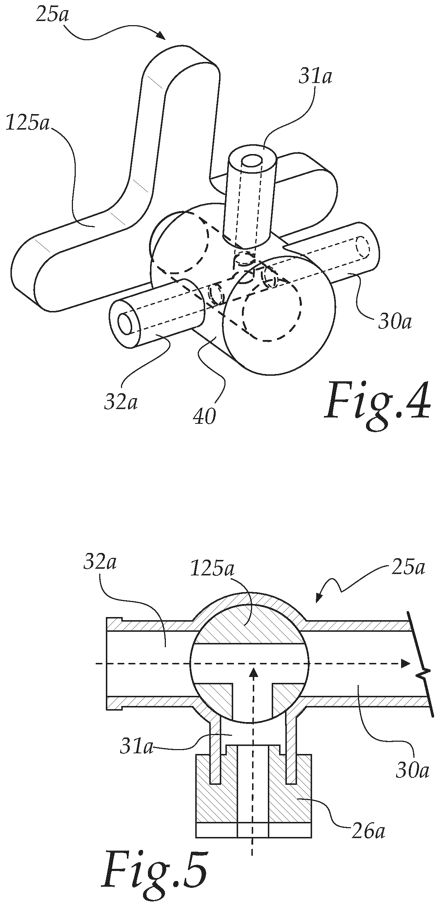

Figures 3 to 5 show, in particular, the valve 25a; the valve 25b is similar to it.

As can be seen in these figures, it has a valve body which comprises a central chamber 50 which intersects three portions 30a, 31a, 32a or 30b, 31b, 32b, respectively a first portion 30a or 30b, a second portion 31a or 31b and a third portion 32a or 32b.

The longitudinal axes along which the portions 30a, 31a, 32a or 30b, 31b, 32b are oriented are mutually coplanar and perpendicular, and are also perpendicular to the axis of longitudinal extension of the chamber 50.

Each one of the valves 25a and 25b can be inserted internally in the appendages 17a and 17b with an orientation which is variable according to the requirements of use.

In particular, the longitudinal axes of the portions 30a, 31a, 32a and 30b, 31b, 32b can be substantially coplanar with the plane of extension of the handpiece 11 or, alternatively, lie on planes that are substantially mutually parallel and incident to the plane of arrangement of the handpiece 11.

Also, alternatively, the valves 25a and 25b can be inserted in such a manner that the longitudinal axes of the portions 30a, 31a and 32a and the portions 30b, 31b and 32b lie on planes that are mutually incident and also incident to the plane of arrangement of the handpiece 11.

As can be seen in Figure 2, the end portion 40a or 40b is fitted externally onto the first portion 30a or 30b.

The third portion 32a or 32b is advantageously and substantially coaxial with respect to the first portion 30a or 30b, both being oriented substantially and coaxially with the flexible appendage 17a or 17b.

The second portion 31a or 31b on the other hand is perpendicular to the portions 30a, 32a or 30b, 32b.

Such a shape structure of the valves 25a and 25b makes it possible to obtain overall:
- two lines for the insertion of one or more surgical or diagnostic instruments, represented by the third portions 32a and 32b; and
- two lines for connection to a system for dispensing fluids, represented by the second portions 31a and 31b.

In this manner, the device 10 can simultaneously receive two surgical and/or diagnostic instruments, respectively inserted into the device 10 via the first valve 25a and via the second valve 25b.

In the particular case of the embodiment of the protective device 10 according to the invention, two instruments with a diameter of 5 Fr/Ch (i.e. substantially 1.65 mm) can be inserted and therefore used at the same time, or one instrument with a diameter of 5 Fr/Ch and one with a diameter of 7 Fr/Ch (i.e. substantially 2.10 mm).

Alternatively, the device 10 can still receive a single surgical and/or diagnostic instrument, inserted indifferently via the portion 32a or 32b of the first valve 25a or of the second valve 25b according to requirements, owing for example to the fact that the healthcare operator who performs the examination and/or the surgery is right-handed or left-handed.

In this case, the third portion 32a or 32b is advantageously closed with a sealing element 26a or 26b, more specifically and preferably a standardized "Luer lock" plug.

Differently, the second portions 31a and 31b are adapted to be connected to a system for administering washing fluids, which can be irrigated inside the cavity of the human body targeted by the examination and/or surgery, or extracted from it.

The flow of the washing fluid irrigated and/or extracted can be regulated by a flow control element 125a or 125b of the valve 25a or 25b, which has a plurality of openings which can be associated with the portions 30a, 31a and 32a and 30b, 31b and 32b, by means of a rotation of the flow control element 125a or 125b about an axis that substantially coincides with the axis of longitudinal extension of the chamber 50.

Such a rotation makes it possible to align, at the discretion of the operator, the openings of the flow control element 125a with at least some of the portions 30a, 31a and 32a or 30b, 31b and 32b, in such a manner as to allow and/or prevent the passage of a washing fluid through preferential routes.

In particular, with the invention, in addition to and at the same time as the insertion of two surgical and/or diagnostic instruments, it is possible at the same time to perform the extraction and irrigation of the washing fluid by way of the connection of a system for administering fluids with the second portions 31a and 31b.

The irrigation does not have its own internal dedicated conduit, but is conducted at the same time as the extraction and in the same conduit; this is possible because a washing fluid that is irrigated in the cavity to be examined and/or operated on initially has certain chemical/physical properties, which are altered when the washing fluid comes into contact with the cavity.

The variability of the properties of the fluid makes it possible to distinguish, even though it is the same fluid under different conditions:
- a first fluid, which has the initial conditions before coming into contact with the cavity,
- a second fluid, which is the same washing fluid but on its way out, after having come into contact with the cavity.

The chemical/physical properties of the washing fluid include, in particular, the pH, the density, the viscosity, the temperature and the pressure.

Furthermore, even the forces of gravity to which the first and the second fluid are subjected are different, in that generally the sac containing the washing liquid is placed further away from the ground with respect to the cavity targeted by the examination and/or by the surgery.

The washing liquid used is usually a substantially 5% saline solution; once it comes into contact with the tissues of the targeted cavity, these tissues partially absorb the salinity, altering its level and varying the pH with respect to the value on entry to the cavity.

The absorption of the salts dissolved in the saline solution also determines a change in the density and in the viscosity of the fluid.

The saline solution, introduced at ambient temperature (usually comprised between 22°C and 24°C), comes into contact with the internal cavity which is substantially at 37-38°C, for sufficient time to alter its temperature.

In order to remove the washing liquid once it has been used inside the cavity, it is furthermore necessary for the region where the fluid is introduced and the region where it is extracted to have different pressures, so determining a consequent change in pressure of the washing liquid.

The different conditions in which the first fluid and the second fluid find themselves, i.e. the same fluid before and after coming into contact with the internal cavity, make it possible to conduct simultaneously the irrigation and the extraction of the first fluid and of the second fluid when these are inside the same conduit, defined by the interspace between the inner wall of the cannula and the optical probe.

The different properties of the two fluids create a form of thin fluid membrane which substantially prevents their mixing.

Furthermore, the insertion of instruments into the device 10 creates a kind of separation wall inside the conduit, identified by the interspace, which subdivides it into two lines; in particular, the insertion of such instruments consists in a mechanical means of division of the first and second fluid, supporting the separation made possible by their different chemical/physical properties.

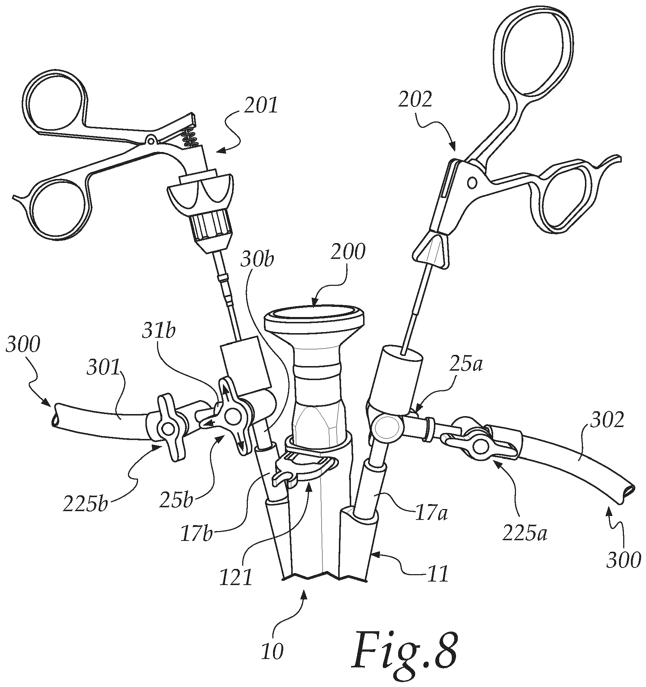

Figure 8 shows in more detail the device 10 in a configuration of use thereof, in which two surgical instruments, identified by the reference numerals 201 and 202, and the optical probe, identified by the reference numeral 200, are inserted.

Furthermore, as can be seen, the device 10 is connected to a system 300 for administering fluids; more specifically, two further two-way valves 225a and 225b are used to connect the valves 25a and 25b to the extraction and irrigation conduits of the washing liquid, respectively identified by the reference numerals 301 and 302.

The two further valves 225a and 225b serve substantially to intercept and/or in any case regulate the flow of the washing fluid.

The same figure also shows how each one of the valves 25a and 25b can be oriented differently with respect to the flexible appendages 17a and 17b according to requirements.

The use of the protective device according to the invention is evident from the foregoing description and explanation and, in particular, it is evident that, by virtue of the insertion of one or two three-way valves 25a and 25b in the flexible appendages 17a and 17b of the device, it is possible to obtain an instrument that allows the simultaneous insertion of two surgical instruments and the simultaneous extraction/irrigation of a washing fluid.

In practice it has been found that the invention fully achieves the intended aim and objects by providing a single-use protective device that enables a healthcare operator to perform diagnostic examinations and/or surgical operations more rapidly and more easily.

In fact, an advantage of the invention is to provide a protective device that allows the simultaneous insertion of multiple surgical instruments.

Another advantage of the invention is that it provides a protective device that enables the simultaneous extraction and irrigation of a washing fluid in the cavity affected by the medical examination and/or by the surgical operation, even simultaneously with the insertion of multiple surgical instruments.

The invention thus conceived is susceptible of numerous modifications and variations, all of which are within the scope of the appended claims. Moreover, all the details may be substituted by other, technically equivalent elements.

In practice the materials employed, provided they are compatible with the specific use, and the contingent dimensions and shapes, may be any according to requirements and to the state of the art.

The disclosures in Italian Patent Application No. 102024000008575 from which this application claims priority are incorporated herein by reference.

Where technical features mentioned in any claim are followed by reference signs, such reference signs have been inserted for the sole purpose of increasing the intelligibility of the claims and accordingly such reference signs do not have any limiting effect on the interpretation of each element identified by way of example by such reference signs.

## Claims

1. A protective device (10) for an endoscopic apparatus comprising a handpiece (11) made of plastic material from which a self-supporting, rigid cannula (12) made of metallic material protrudes, said handpiece (11) comprising two tubular and flexible appendages (17a, 17b) which converge into a primary conduit (24) which passes through said handpiece (11) internally,
**characterized in that** it comprises at least one three-way valve (25a, 25b) engaged with at least one of said flexible tubular appendages (17a, 17b).

2. The device (10) according to claim 1, **characterized in that** it comprises two three-way valves (25a, 25b), respectively a first three-way valve (25a) and a second three-way valve (25b), each one engaged with one of said flexible tubular appendages (17a, 17b).

3. The device (10) according to claim 1, **characterized in that** said at least one three-way valve (25a, 25b) is inserted with interference into an end portion (40a, 40b) of said flexible appendages (17a, 17b) which is opposite with respect to the end inserted in said handpiece (11), said at least one three-way valve (25a, 25b) being insertable with an orientation which is variable according to the requirements of use.

4. The device (10) according to claim 1, **characterized in that** the material of said flexible tubular appendages (17a, 17b) and the material of said at least one three-way valve (25a, 25b) are such as to provide a hydraulically and mechanically tight coupling.

5. The device (10) according to one or more of the preceding claims, **characterized in that** said at least one three-way valve (25a, 25b) has a valve body which comprises a central chamber (50) which intersects at least three portions (30a, 31a, 32a, 30b, 31b, 32b), respectively, at least one first portion (30a, 30b), at least one second portion (31a, 31b) and at least one third portion (32a, 32b), said at least three portions being oriented along mutually coplanar and perpendicular axes and also being substantially perpendicular to the axis of longitudinal extension of said chamber (50), said at least one first portion (30a, 30b) being substantially coaxial with said at least one third portion (32a, 32b) and said at least one second portion (31a, 31b) being substantially perpendicular to said at least one first portion (30a, 30b) and said at least one third portion (32a, 32b).

6. The device (10) according to one or more of the preceding claims, **characterized in that** said at least one three-way valve (25a, 25b) comprises a flow control element (125a) having a plurality of openings which can be associated with said portions (30a, 31a, 32a, 30b, 31b, 32b) by means of a rotation of the flow control element (125a, 125b) about an axis that substantially coincides with the axis of longitudinal extension of said chamber (50), so as to align said openings of said flow control element (125a) with at least some of said portions (30a, 31a, 32a, 30b, 31b, 32b).

7. The device (10) according to claim 5, **characterized in that** it comprises sealing elements (26a, 26b) for closing at least one of said at least three portions (30a, 31a, 32a, 30b, 31b, 32b).

8. The device (10) according to claim 5, **characterized in that** said at least one second portion (31a, 31b) is adapted to be connected to a system (300) for administering fluids.

9. The device (10) according to claim 5, **characterized in that** said at least one third portion (32a, 32b) is adapted to serve as an inlet for the insertion of one or more surgical and/or diagnostic instruments (201, 202).

10. The device (10) according to claim 1, **characterized in that** said handpiece (11) has an upper opening (111) at an end portion (100) of said handpiece (11) that is opposite the one from which said cannula (12) protrudes, said opening (111) being defined correspondingly to an end element (13) and having an elongated shape extending in a direction substantially perpendicular to the longitudinal direction of extension of said handpiece (11), said end portion (13) being geometrically shaped for its form-fit coupling with a U-shaped element (121) adapted to be fitted externally onto said end portion (13) through said opening (111).
